# EUROPÄISCHE PATENTSCHRIFT

(11) **EP 0 648 753 B1**
(45) Veröffentlichungstag und Bekanntmachung des Hinweises auf die Patenterteilung: **02.04.1997**
(21) Anmeldenummer: 94810540.8
(22) Anmeldetag: 20.09.1994
(51) Int. Cl.: C07D 251/24, C08K 5/3492

(54) **Verfahren zur Herstellung von 1,3,5-Triazinen**
Process for the production of 1,3,5-triazines
Procédé pour la préparation des 1,3,5-triazines

(30) Priorität: 28.09.1993 CH 2913/93
(43) Veröffentlichungstag der Anmeldung: 19.04.1995
(73) Patentinhaber: Ciba Specialty Chemicals Holding Inc., 4057 Basel (CH)
(72) Erfinder: Reinehr, Dieter, Dr., D-79400 Kandern (DE); Borsos, Elek, CH-4127 Birsfelden (CH)

(56) Entgegenhaltungen:
- WO-A-94/05645
- CH-A- 480 090
- DE-A- 549 969
- GB-A- 1 518 836
- US-A- 3 113 942

## Beschreibung

Die vorliegende Erfindung betrifft ein einfaches Verfahren zur Herstellung von hydroxyphenylsubstituierten 1,3,5-Triazinen, ausgehend von gegebenenfalls substituierten o-Hydroxybenzonitrilen und gegebenenfalls substituierten Benzonitrilen.

Die Herstellung von symmetrischen, dreifach hydroxyphenylsubstituierten 1,3,5-Triazinverbindungen durch Trimerisierung von aromatischen Hydroxynitrilverbindungen ist bekannt, z.B. aus der US-A-3,113,942.

Die Herstellung von unsymmetrisch substituierten Triazinverbindungen durch Reaktion zweier verschiedener Nitrilverbindungen ist aus der DRP 549969 bekannt. In diesem Verfahren wird die Reaktion in Anwesenheit von Chlorsulfonsäure durchgeführt. Die Verwendung von hydroxy-substituierten Arylcyaniden für diese Reaktion ist nicht bekannt.

Es wurde nun gefunden, dass hydroxysubstituierte 1,3,5-Triazinverbindungen auf einfache Weise, ausgehend von gegebenenfalls substituierten o-Hydroxybenzonitrilen, hergestellt werden können.

Die vorliegende Erfindung betrifft daher ein Verfahren zur Herstellung von Triazinverbindungen der Formel 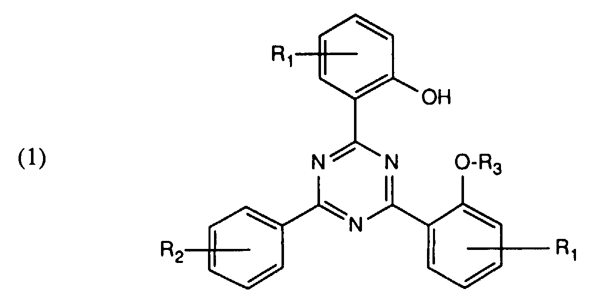 wobei man im ersten Reaktionsschritt ein Benzonitril der Formel 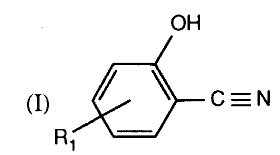 mit einem Nitril der Formel (II) 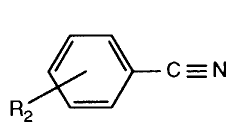 zur Verbindung der Formel 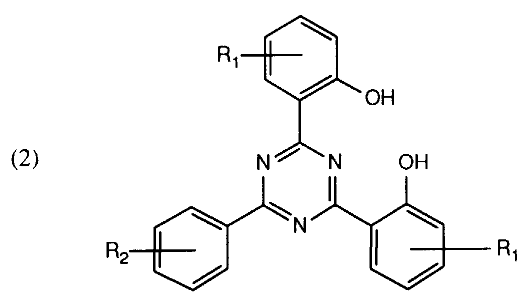 umsetzt und im zweiten Reaktionsschritt diese Verbindung durch selektive Alkylierung zur Verbindung der Formel (1) bei einer Temperatur von 20 bis 170°C umsetzt,
worin
- R₁ und R₂: unabhängig voneinander Wasserstoff, Halogen, -C≡N, C₁-C₁₈-Alkyl oder C₁-C₁₈-Alkoxy und
- R₃: C₁-C₁₈-Alkyl
bedeuten.

C₁-C₁₈-Alkyl und C₁-C₁₈-Alkoxy sind geradkettige oder verzweigte Alkyl- bzw. Alkoxyreste wie z.B. Methyl, Ethyl, n-Propyl, Isopropyl, n-Butyl, sek.Butyl, tert.Butyl, Amyl, Isoamyl oder tert.Amyl, Heptyl, Octyl, Isooctyl, Nonyl, Undecyl, Dodecyl, Tetradecyl, Pantadecyl, Hexadecyl, Heptadecyl oder Octadecyl bzw. Methoxy, Ethoxy, Propoxy, Butoxy, Pentyloxy, Hexyloxy, Heptyloxy, Octyloxy, Isooctyloxy, Nonyloxy, Undecyloxy, Dodecyloxy, Tetradecyloxy, Pentadecyloxy, Hexadecyloxy, Heptadecyloxy oder Octadecyloxy.

Halogen bedeutet Chlor, Brom oder Iod. Chlor ist bevorzugt.

Als Alkylierungsreagenzien kommen Alkylhalogenide, Dialkylsulfate, Toluolsulfonate oder Dialkylalkanphosphonate in Betracht. Vorzugsweise verwendet man Dialkylalkanphosphonate oder Dialkylsulfat, besonders Dimethylalkanphosphonat oder Dimethylsulfat.

Für das erfindungsgemässe Herstellungsverfahren kommen vorzugsweise Verbindungen der Formel (1) in Betracht, bei denen
R₁und R₂ jeweils Wasserstoff und
R₃ C₁-C₁₈-Alkoxy
bedeuten.

Die Reaktionszeit für den ersten Reaktionsschritt beträgt in der Regel 2 bis 24 Stunden, vorzugsweise 5 bis 12 Stunden. Lösungsmittel und weitere Zusätze sind für die Umsetzung nicht notwendig. Auch auf die Verwendung von Katalysatoren kann verzichtet werden.

Die bevorzugte Reaktionstemperatur liegt zwischen 180° und 260°C.

Das Molverhältnis der eingesetzten Nitrilverbindungen der Formeln (I) und (II) kann über einen breiten Bereich variiert werden. Vorzugsweise beträgt das Molverhältnis der Nitrilverbindungen der Formeln (I) und (II) 2:1 bis 1:10.

Die Durchführung des ersten Reaktionsschrittes erfolgt im allgemeinen so, dass man die Ausgangsverbindungen der Formeln (II) und (III) unter Rühren zusammenbringt, bis zur vollständigen Schmelze der Reaktanden erhitzt und bei dieser Temperatur während der angegebenen Reaktionszeit weiterrührt.

Die Alkylierungsreaktion (zweiter Reaktionsschritt) wird im alkalischen Medium unter Verwendung von Natrium- oder Kaliumcarbonat oder verdünnter Natrium- oder Kaliumhydroxid-Lösung durchgeführt. In der Praxis geht man z.B. so vor, dass man die Ausgangsverbindung, Alkalicarbonat bzw. Natriumhydroxid-Lösung zusammen mit dem Alkylierungsreagenz mischt und bei einer Temperatur von 20 bis 170°C über einen Zeitraum von 1 bis 7 Stunden rührt. Ein weiteres Lösungsmittel ist für die Reaktion nicht notwendig. Verwendet man als Alkylierungsreagenz Dialkylalkanphosphonat, wird normalerweise wasserfrei gearbeitet. Nach gewöhnlicher Aufarbeitung erhält man das Produkt entsprechend Formel (1) in guten Ausbeuten und hoher Reinheit.

Die vorliegende Erfindung betrifft auch ein Verfahren zur Herstellung von zweifach hydroxyphenylsubstituierten 1,3,5-Triazinverbindungen der Formel (2). Das Verfahren ist dadurch gekennzeichnet, dass man ein Benzonitril der Formel (I) mit einem Benzonitril der Formel (II) bei einer Temperatur von 180 bis 260°C, worin
R₁ und R₂ die angegebenen Bedeutungen haben, zur Verbindung der Formel (2) umsetzt.

Mit diesem Verfahren lassen sich zweifach hydroxyphenylsubstituierte 1,3,5-Triazinverbindungen auf einfache Weise und in guten Ausbeuten herstellen.

Die nach den erfindungsgemässen Verfahren hergestellten Triazin-Verbindungen finden Verwendung als UV-Absorber oder sind Ausgangsprodukte für die Herstellung von UV-Absorbern.

Die folgenden Beispiele veranschaulichen die Erfindung.

Beispiel 1:
35,2 g (0,3 Mol) p-Tolunitril werden zusammen mit 11,9 g (0,1 Mol) 2-Hydroxybenzonitril vorgelegt und unter Rühren auf eine Innentemperatur von 217°C gebracht. Die klare rötliche Schmelze wird während 6 Stunden bei 217-222°C gerührt. Dann wird auf 80°C abgekühlt und mit 20 ml Ethanol versetzt. Es entsteht eine gut rührbare Suspension, die auf Raumtemperatur abgekühlt und abfiltriert wird. Nach dem Waschen mit mehreren Portionen Ethanol und Wasser wird im Vakuum bei 80°C getrocknet. Man erhält 10,5 g eines hellen Pulvers der Formel 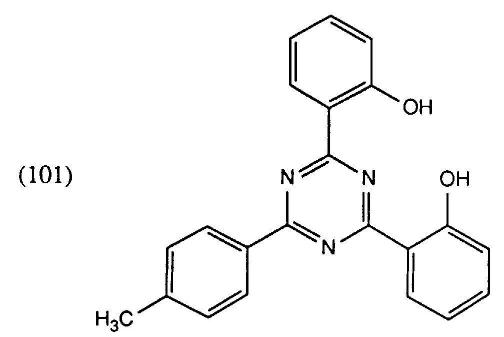 entsprechend einer Ausbeute von 59,2% d.Th. mit einem Schmelzpunkt von 258-259°C.

Das Produkt ist noch mit etwas Tris(-o-hydroxyphenyl)-1,3,5-triazin (Verbindung der Formel (103)) verunreinigt. Der Schmelzpunkt des umkristallisieten Produktes beträgt 258-259°C.

Beispiel 2: Verwendet man 93,6 g (0,8 Mol) p-Tolunitril und 119 g (1 Mol) 2-Hydroxybenzonitril während 15 Stunden bei 210-217°C, so erhält man 138,5 g der Verbindung der Formel (101), was einer Ausbeute von 77,9% d.Th. entspricht.

Beispiel 3: Man verfährt wie in Beispiel 1 angegeben, mit dem Unterschied, dass man 20,6 g (0,2 Mol) Benzonitril anstelle von 35,2 g Tolunitril verwendet. Nach einer Reaktionszeit von 6 Stunden bei 190 bis 195°C erhält man 10,4 g eines Gemisches der Verbindung der Formel 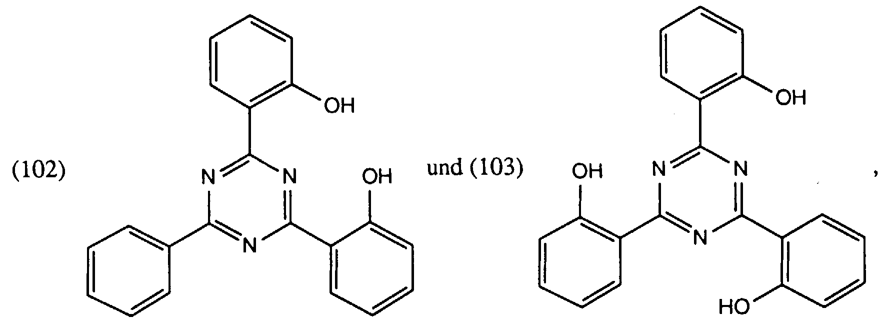 das aber überwiegend aus der Verbindung der Formel (102) besteht, entsprechend einer Ausbeute von 60,9 % d.Th.

Beispiel 4: Man verfährt wie in Beispiel 3 beschrieben mit dem Unterschied, dass man 35,7 g (0,3 Mol) 2-Hydroxybenzonitril und 30,9 g (0,3 Mol) Benzonitril verwendet. Nach einer Reaktionszeit von 6 Stunden bei 250°C im Stahlautoklaven erhält man nach der Aufarbeitung 41,9 g eines Produktes, das überwiegend aus der Verbindung der Formel (102) besteht, entsprechend einer Ausbeute von 81,8 % d.Th.

Beispiel 5: Man verfährt wie in Beispiel 1 beschrieben, mit dem Unterschied, dass man 79,9 g (0,6 Mol) 4-Methoxybenzonitril (anstelle von 35,2 g p-Tolunitril) und 23,8 g (0,2 Mol) (anstelle von 11,9 g) 2-Hydroxybenzonitril verwendet. Nach einer Reaktionszeit von 8 Stunden bei Temperaturen von 245 bis 260°C erhält man nach der Aufarbeitung 14,9 g (40,1% d. Th.) der Verbindung der Formel

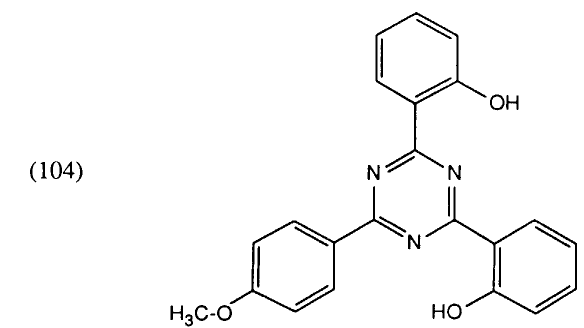

Beispiel 6: Die Verbindung der Formel (101) wird mit Methan-phosphonsäure-dimethylester und Soda als Base bei 150°C und nach einer Reaktionszeit von 4 Stunden in die Verbindung der Formel 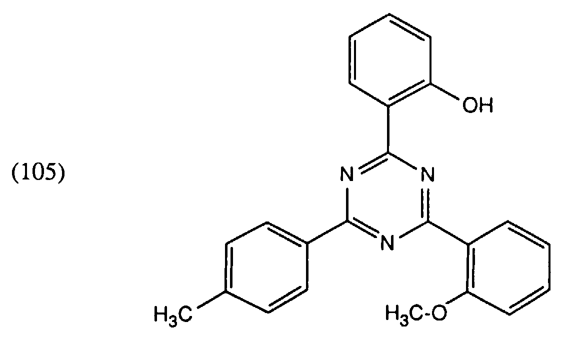 überführt.

Smp.: 168-169°C

Beispiel 7: Die Verbindung der Formel (102) wird, wie in Beispiel 6 beschrieben, in die Verbindung der Formel 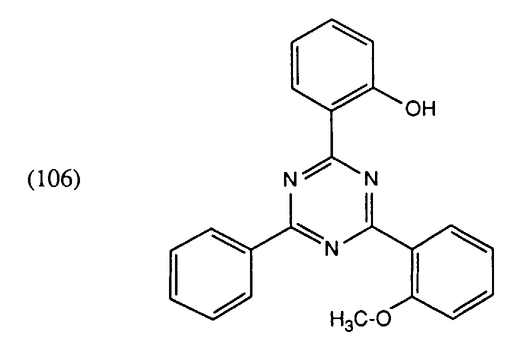 überführt.

Schmelzpunkt: 153-155°C.

Beispiel 8: Die Verbindung der Formel (104) wird, wie in Beispiel 6 beschrieben, in die Verbindung der Formel 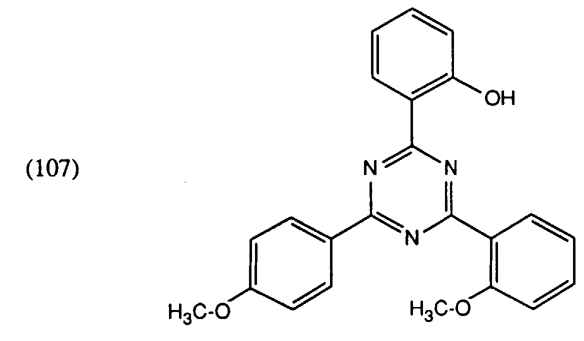 überführt.

Schmelzpunkt: 152-153°C.

## Patentansprüche

1. Verfahren zur Herstellung einer Triazinverbindung der Formel 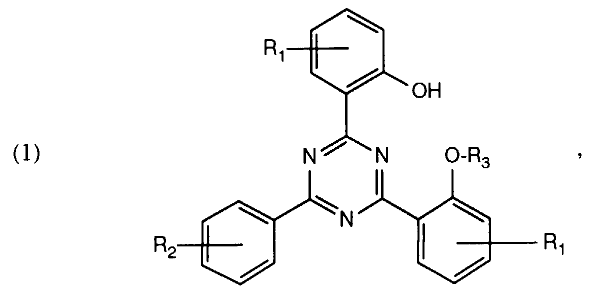 durch Umsetzung im ersten Reaktionsschritt eines Benzonitrils der Formel 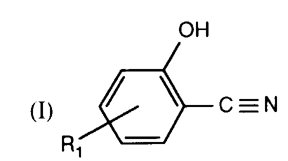 mit einem Nitril der Formel (II) 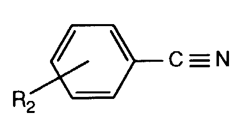 zur Verbindung der Formel 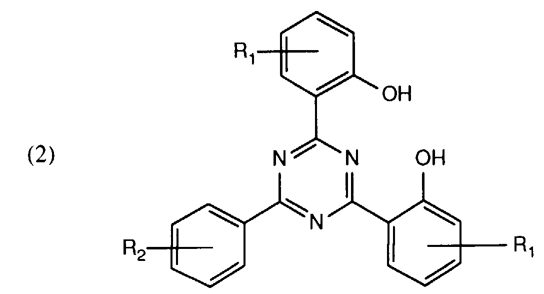 und im zweiten Reaktionsschritt durch selektive Alkylierung dieser Verbindung bei 20-170°C zur Verbindung der Formel (1),
worin
R₁ und R₂ unabhängig voneinander Wasserstoff, Halogen, -C≡N, C₁-C₁₈-Alkyl oder C₁-C₁₈-Alkoxy und
R₃ C₁-C₁₈-Alkyl
bedeuten.

2. Verfahren nach Anspruch 1, dadurch gekennzeichnet, dass man als Alkylierungsreagenz ein Dialkyl-alkanphosphonat oder Dialkylsulfat verwendet.

3. Verfahren nach Anspruch 1 oder 2, dadurch gekennzeichnet, dass man als Alkylierungsreagenz Dimethylalkanphosphonat oder Dimethylsulfat verwendet.

4. Verfahren nach einem der Ansprüche 1 bis 3, dadurch gekennzeichnet, dass die Reaktion der ersten Stufe bei einer Temperatur von 180 bis 260°C durchgeführt wird.

5. Verfahren nach einem der Ansprüche 1 bis 4, dadurch gekennzeichnet, dass das Molverhältnis der Benzonitrile der Formeln (I) und (II) 2:1 bis 1:10 beträgt.

6. Verfahren nach Anspruch 4, dadurch gekennzeichnet, dass Verbindungen der Formel (1) hergestellt werden, worin
R₁ und R₂ jeweils Wasserstoff und
R₃ C₁-C₁₈-Alkoxy
bedeuten.

7. Verfahren nach einem der Ansprüche 1 bis 6, dadurch gekennzeichnet, dass die Reaktionszeit in der ersten Stufe 2 bis 24 Stunden, vorzugsweise 5 bis 12 Stunden, beträgt.

8. Verfahren nach einem der Ansprüche 1 oder 7, dadurch gekennzeichnet, dass die Umsetzung in der ersten Stufe lösungsmittelfrei durchgeführt wird.

9. Verfahren nach einem der Ansprüche 1 bis 8, dadurch gekennzeichnet, dass die Umsetzung in der ersten Stufe ohne die Verwendung von Katalysatoren durchgeführt wird.

10. Verfahren zur Herstellung von Triazinverbindungen der Formel 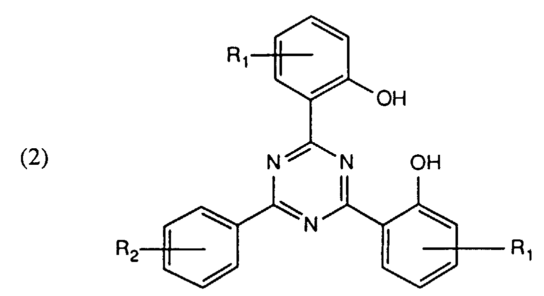 durch Umsetzung eines Benzonitrils der Formel (I) 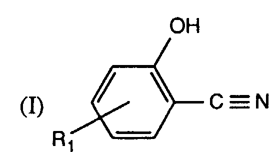 mit einem Benzonitril der Formel (II) 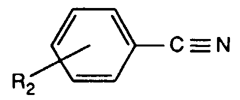 bei einer Temperatur von 180 bis 260°C,
worin
R₁ und R₂ unabhängig voneinander Wasserstoff, Halogen, -C≡N, C₁-C₁₈-Alkyl oder C₁-C₁₈-Alkoxy bedeuten.

11. Verwendung der nach den Ansprüchen 1 bis 10 hergestellten Verbindungen als UV-Absorber oder als Ausgangsprodukte für die Herstellung von UV-Absorbern.

## Claims

1. A process for the preparation of a triazine compound of formula 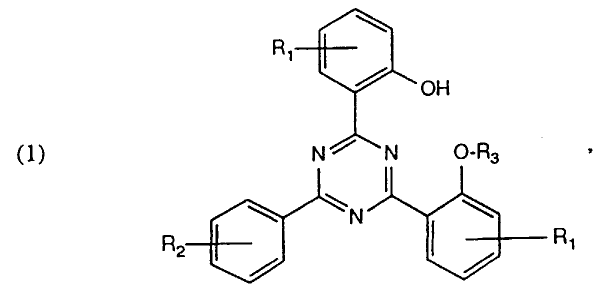 by, in a first reaction step, reacting a benzonitrile of formula 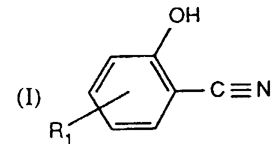 with a nitrile of formula (II) 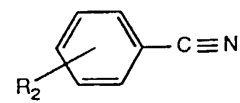 to the compound of formula 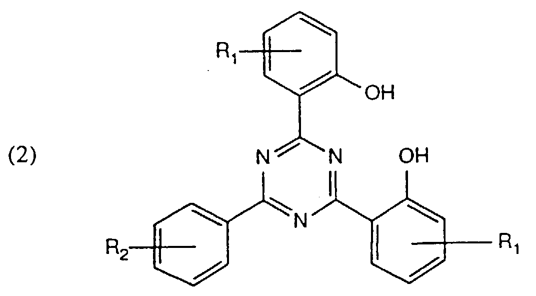 and, in a second reaction step, reacting said compound by selective alkylation at 20-170°C to the compound of formula (1),
in which
R₁ and R₂ are each independently of the other hydrogen, halogen, -C≡N,C₁-C₁₈alkyl or C₁-C₁₈alkoxy and
R₃ is C₁-C₁₈alkyl.

2. A process according to claim 1, wherein the alkylating reagent used is a dialkyl alkanephosphonate or dialkyl sulfate.

3. A process according to claim 1 or 2, wherein the alkylating reagent used is dimethyl alkanephosphonate or dimethyl sulfate.

4. A process according to any one of claims 1 to 3, wherein the reaction of the first stage is carried out at a temperature from 180 to 260°C.

5. A process according to any one of claims 1 to 4, wherein the molar ratio of the benzonitriles of formulae (I) and (II) is from 2:1 to 1:10.

6. A process according to claim 4, wherein compounds of formula (1), are prepared in which
R₁ and R₂ are each hydrogen and
R₃ is C₁-C₁₈alkoxy.

7. A process according to any one of claims 1 to 6, wherein the reaction time in the first stage is from 2 to 24 hours, preferably from 5 to 12 hours.

8. A process according to any one of claims 1 to 7, wherein the reaction in the first stage is carried out without a solvent.

9. A process according to any one of claims 1 to 8, wherein the reaction in the first stage is carried out without the use of catalysts.

10. A process for the preparation of triazine compounds of formula 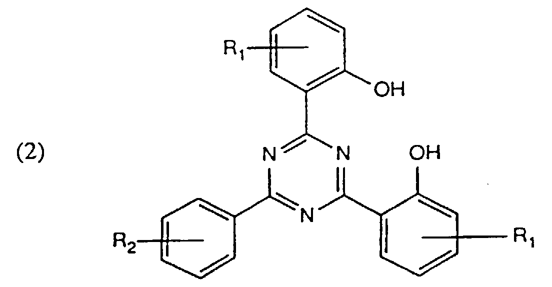 by reacting a benzonitrile of formula 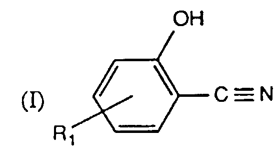 with a benzonitrile of formula (II) 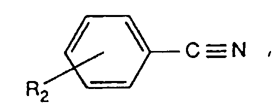 at a temperature from 180 to 260°C,
in which
R₁ and R₂ are each independently of the other hydrogen, halogen, -C≡N, Cₗ-C₁₈alkyl or C₁-C₁₈alkoxy.

11. Use of a compound prepared according to any one of claims 1 to 10 as UV absorber or as starting material for the preparation of UV absorbers.

## Revendications

1. Procédé pour la préparation d'un composé de triazine de formule 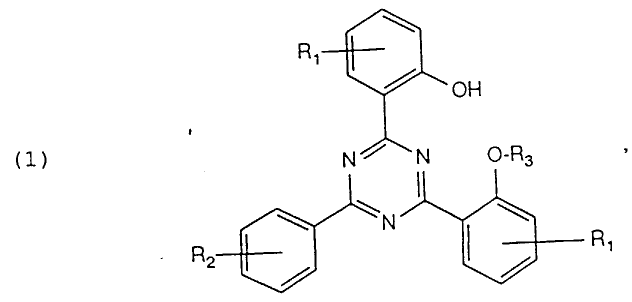 par réaction, dans une première étape réactionnelle, d'un benzonitrile de formule (I) 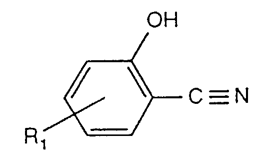 avec un nitrile de formule (II) 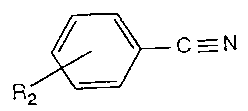 pour obtenir le composé de formule 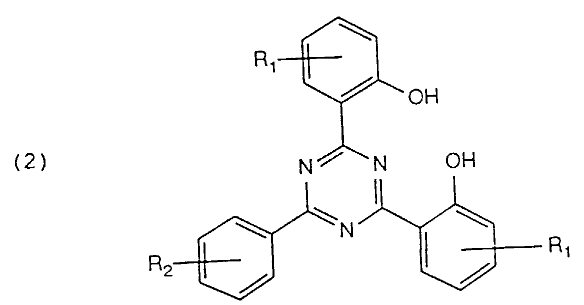 et, dans une deuxième étape réactionnelle, par alkylation sélective de ce composé, à une température de 20 à 170 °C, pour obtenir le composé de formule (1),
où
R₁ et R₂, indépendamment l'un de l'autre, représentent l'hydrogène, halogène, -C≡N, alkyle en C₁-C₁₈ ou alcoxy en C₁-C₁₈ et
R₃ représente alkyle en C₁-C₁₈.

2. Procédé selon la revendication 1, caractérisé en ce que l'on utilise comme réactif d'alkylation un dialkylalcanephosphonate ou un sulfate de dialkyle.

3. Procédé selon la revendication 1 ou 2, caractérisé en ce que l'on utilise comme réactif d'alkylation un diméthylalcanephosphonate ou le sulfate de diméthyle.

4. Procédé selon l'une des revendications 1 à 3, caractérisé en ce que l'on met en oeuvre la réaction dans une première étape à une température de 180 à 260 °C.

5. Procédé selon l'une des revendications 1 à 4, caractérisé en ce que le rapport en moles du benzonitrile de formules (I) et (II) est de 2:1 à 1:10.

6. Procédé selon la revendication 4, caractérisé en ce que l'on prépare des composés de formule (1) dans lesquels
R₁ et R₂ représentent chacun l'hydrogène, et
R₃ représente alcoxy en C₁-C₁₈.

7. Procédé selon l'une des revendications 1 à 6, caractérisé en ce que la durée de réaction dans la première étape est de 2 à 24 heures, de préférence de 5 à 12 heures.

8. Procédé selon l'une des revendications 1 ou 7, caractérisé en ce que la réaction est mise en oeuvre dans la première étape sans solvant.

9. Procédé selon l'une des revendications 1 à 8, caractérisé en ce que l'on met en oeuvre la réaction dans la première étape sans utilisation de catalyseurs.

10. Procédé pour la préparation des composés de triazine de formule 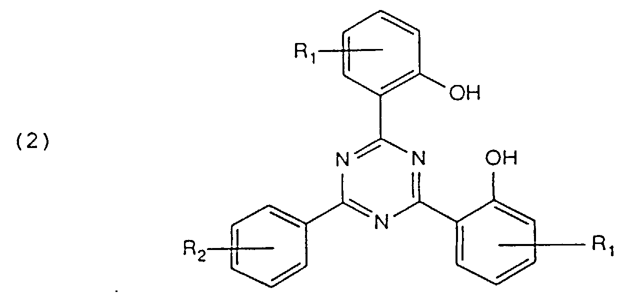 par réaction d'un benzonitrile de formule (I) 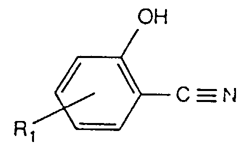 avec un benzonitrile de formule (II) 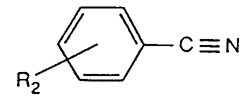 à une température de 180 à 260 °C, où
R₁ et R₂, indépendamment l'un de l'autre, représentent l'hydrogène, halogène, -C≡N, alkyle en C₁-C₁₈ ou alcoxy en C₁-C₁₈.

11. Utilisation des composés préparés selon les revendications 1 à 10, comme absorbeurs d'UV ou comme produits de départ pour la préparation des absorbeurs d'UV.
